# EUROPÄISCHE PATENTSCHRIFT

(11) **EP 2 720 732 B1**
(45) Veröffentlichungstag und Bekanntmachung des Hinweises auf die Patenterteilung: **05.08.2015**
(21) Anmeldenummer: 12727798.6
(22) Anmeldetag: 11.06.2012
(51) Int. Cl.: A61M 1/16, A61M 5/44

(54) **VORRICHTUNG ZUR ERWÄRMUNG EINER MEDIZINISCHEN FLÜSSIGKEIT**
DEVICE FOR HEATING A MEDICAL LIQUID
DISPOSITIF POUR CHAUFFER UN LIQUIDE MÉDICINAL

(30) Priorität: 15.06.2011 DE 102011104218; 15.06.2011 US 201161497149 P
(43) Veröffentlichungstag der Anmeldung: 23.04.2014
(73) Patentinhaber: Fresenius Medical Care Deutschland GmbH, 61352 Bad Homburg (DE)
(72) Erfinder: WIKTOR, Christoph, 63571 Gelnhausen (DE)
(74) Vertreter: Laufhütte, Dieter
(86) Internationale Anmeldenummer: PCT/EP2012/002467
(87) Internationale Veröffentlichungsnummer: WO 2012/171629

(56) Entgegenhaltungen:
- EP-B1- 0 254 801
- WO-A2-03/099354
- WO-A2-2007/084703
- US-A1- 2005 008 354
- US-A1- 2009 012 655

## Beschreibung

Die vorliegende Erfindung betrifft eine Vorrichtung zur Erwärmung einer medizinischen Flüssigkeit, ein Verfahren zur Erwärmung einer medizinischen Flüssigkeit sowie eine Blutbehandlungsvorrichtung.

In der extrakorporalen Blutbehandlung ist es notwendig, die prozessierten Flüssigkeiten zu temperieren, um ein Auskühlen des Patienten zu verhindern. In der Regel werden bei den extrakorporalen Dialysebehandlungen des Bluts die Dialysierflüssigkeit, oder weitere Flüssigkeiten, wie z. B. Substitutionsflüssigkeiten, auf eine entsprechende Temperatur gebracht, um das Blut im extrakorporalen Kreislauf nicht zu sehr auskühlen zu lassen.

Temperierungen von Dialysierflüssigkeiten finden sich auch in Behandlungssystemen der Peritonealdialyse. Die Dialysierflüssigkeit wird dabei dem Patienten in das Peritoneum verabreicht und muss entsprechend temperiert werden. Dabei sind verschiedene Behandlungsmodi bekannt, bei denen die Dialysierflüssigkeit nach automatisierten Flussschemen dem Patienten zugeführt werden und die Dialysierflüssigkeit entsprechend dem Flussschema temperiert werden muss.

In der Hämodialyse wie auch in der Peritonealdialyse sind Systeme in Entwicklung und bereits Stand der Technik, bei denen hydraulische und thermische Funktions-elemente zur Verarbeitung der Dialysierflüssigkeit oder andere physiologische Flüssigkeiten, wie z. B. Substitutionsflüssigkeit (EN) auf Einwegartikeln erfolgt. Die Verwendung von Einwegartikeln, die in der Regel aus Kunststoffen bestehen, stellt besondere Anforderungen an die Regelung der Heizquelle.

Dabei ist zu beachten, dass die Temperaturregelung von Flüssigkeiten in Einwegartikeln ein langsamer Vorgang ist. Durch die schlechte Wärmeübertragung im Einwegartikel sind zudem hohe Temperaturen an der Heizungsschnittstelle notwendig. So ist es keine Seltenheit, dass eine Flüssigkeitstemperatur von 40°C eine Temperatur der Heizplatte von über 80°C erforderlich macht.

Wird der Durchfluss verfahrens- oder alarmbedingt unterbrochen, so steht die Flüssigkeit an der heißen Schnittstelle und kann überhitzt werden. Dies kann dazu führen, dass die Heizung bei Flussstillstand abgeschaltet wird bzw. werden muss. Bei kurzen Stillstandszeiten kann es dennoch zu einer Überhitzung und damit zu einem alarmpflichtigen Zustand kommen. Bei langen Stillstandszeiten kühlt die Flüssigkeit stärker ab.

Die EP 0 956 876 A1 zeigt eine Einwegkassette für die Peritonealdialyse zum Fördern und Temperieren der Dialysierflüssigkeit.

Die US 2009/012655 A1 zeigt eine Vorrichtung gemäß dem Oberbegriff von Anspruch 1, welche ein geregeltes Heizelement zur Temperierung von Dialysierflüssigkeit auf einer Dialysekassette umfasst. Das Heizelement wird dabei auf Basis einer Temperaturabweichung und der Flussrate der Dialysierflüssigkeit angesteuert.

Die EP 0 254 801 B1 zeigt einen Fluiderwärmer mit Mitteln zum Bestimmen der Fluidtemperatur, der Heizertemperatur und der Temperatur einer zwischengeschalteten Heizplatte. Hierbei erfolgt ein Abschalten, wenn Heizer oder Heizplatte zu heiß sind, insbesondere dann, wenn ein stark reduzierter Fluss des Fluids vorliegt.

Es ist daher die Aufgabe der vorliegenden Erfindung, eine Vorrichtung, ein Verfahren sowie eine Blutbehandlungsvorrichtung der eingangs genannten Art in vorteilhafter Weise weiterzubilden, insbesondere dahingehend, dass eine Überhitzung der zu verwendenden und zu erwärmenden medizinischen Flüssigkeit, insbesondere des Dialysates oder sonstige Substitutionslösungen sicher vermieden werden kann.

Diese Aufgabe wird erfindungsgemäß gelöst durch eine Vorrichtung mit dem Merkmal des Anspruchs 1. Danach ist vorgesehen, dass eine Vorrichtung zur Erwärmung einer medizinischen Flüssigkeit, insbesondere einer Dialysierflüssigkeit, wenigstens eine Fluidpassage zur Förderung der medizinischen Flüssigkeit, wenigstens ein Heizelement, mittels dessen die medizinische Flüssigkeit in der Fluidpassage erwärmbar ist, wenigstens ein erstes Erfassungsmittel zum Erfassen eines Parameters, der mit der Flussrate der medizinischen Flüssigkeit durch die Fluidpassage korreliert, und/oder wenigstens ein zweites Erfassungsmittel zum Erfassen eines Parameters, der einem Sollwert für die Flussrate der medizinischen Flüssigkeit durch die Fluidpassage entspricht, wenigstens ein Steuerungs- und/oder Regelungsmittel, mittels dessen die Fluidtemperatur des Fluids in der Fluidpassage und/oder Temperatur des Heizelementes steuer- und/oder regelbar ist, sowie ein Umschaltmittel umfasst, mittels dessen das Steuerungs- und/oder Regelungsmittel von einer Fluidtemperaturregelung auf eine Heizelementtemperaturregelung umschaltbar ist und/oder umgekehrt, wobei das Umschaltmittel derart beschaffen ist, dass eine Umschaltung erfolgt, wenn mittels des Umschaltmittels festgestellt wird, dass ein vorherbestimmter Flussratenschwellwert unter- und/oder überschritten wird.

Es ergibt sich also insbesondere der Vorteil, dass die Temperatur der medizinischen Flüssigkeit auch bei einem Flussstillstand einfach und sicher kontrolliert werden kann. Darüber hinaus erfolgt eine Beschleunigung der Regelung der Flüssigkeitstemperatur nach einem Flussstillstand. Es ist ferner besonders vorteilhaft möglich, eine Verringerung der Untertemperatur der medizinischen Flüssigkeit auch nach langen Stillstandszeiten zu erreichen. Des Weiteren ist es vorteilhaft möglich, eine Vermeidung von alarmpflichtigen Zuständen bei einem Flussstillstand durch Überhitzung zu erreichen.

Eine Fluidtemperaturregelung ist insbesondere eine Regelung der Temperatur des Fluids in der Fluidpassage dahingehend, dass die dortige Temperatur auf einen Sollwert eingeregelt wird.

Eine Heizelementtemperaturregelung ist insbesondere eine Regelung der Temperatur des Heizelements dahingehend, dass die dortige Temperatur auf einen Sollwert eingeregelt wird.

Diese Sollwerte werden jeweils gemäß den jeweiligen Anforderungen gewählt. So kann beispielsweise der Fluidtemperatursollwert in Abhängigkeit von der Flussrate des Fluids durch die Fluidpassage gewählt sein, insbesondere derart gewählt sein, dass das Fluid eine physiologischen Temperatur, z. B. im Bereich zwischen 35°C-37°C aufweist. Dabei kann das Heizelement beispielsweise eine Temperatur von deutlich über 80°C aufweisen.

Der Heizelementtemperatursollwert kann ferner derart gewählt sein, dass auch bei einem Fluidstillstand in der Fluidpassage eine Überhitzung des Fluids sicher vermieden wird. Hierzu kann beispielsweise ein Heizelementtemperatursollwert von nicht über 50°C gewählt werden. Mögliche Temperatursollwerte können vor allem im Temperaturbereich zwischen 30°C und 50° C liegen.

Der vorherbestimmte Flussratenschwellwert, bei dessen Über- oder Unterschreitung eine Umschaltung von einer Fluidtemperaturregelung auf eine Heizelementtemperaturregelung erfolgt, kann beispielsweise eine Flussrate von Null sein. Grundsätzlich ist aber auch möglich, diesen Parameter frei wählen zu können. Denkbar ist insbesondere, den Flussratenschwellwert derart zu wählen, dass bei einem Unterschreiten dieses Schwellwertes eine Umschaltung auf einem Heizelementtempersturregelung erforderlich ist, um eine Überhitzung zu vermeiden.

Des Weiteren kann vorgesehen sein, dass die Vorrichtung weiter wenigstens ein Fördermittel zum Fördern der medizinischen Flüssigkeit durch die Fluidpassage und/oder wenigstens ein erstes Mittel zum Erfassen eines Parameters, der mit der Temperatur des Heizelements korreliert, und/oder wenigstens ein zweites Mittel zum Erfassen eines Parameters, der mit der Temperatur der medizinischen Flüssigkeit in der Fluidpassage korreliert, und/oder wenigstens ein drittes Mittel zum Erfassen eines Parameters, der mit der Flussrate der medizinischen Flüssigkeit durch die Fluidpassage korreliert, und/oder wenigstens ein viertes Mittel zum Erfassen eines Parameters, der einem Sollwert für die Flussrate der medizinischen Flüssigkeit durch die Fluidpassage entspricht, umfasst. Das erste Mittel zum Erfassen eines Parameters, der mit der Temperatur des Heizelements korreliert, und/oder wenigstens ein zweites Mittel zum Erfassen eines Parameters, der mit der Temperatur der medizinischen Flüssigkeit in der Fluidpassage korreliert, können beispielsweise Temperatursensoren sein.

Ferner ist möglich, dass das Steuerüngs- und/oder Regelungsmittel wenigstens ein Kontrollmittel, mittels dessen die Fluidtemperatur anhand eines Parameters, der mit der Fluidtemperatur korreliert, und/oder wenigstens ein zweites Kontrollmittel, mittels dessen die Heizelementtemperatur anhand eines Parameters, der mit der Temperatur des Heizelements korreliert, kontrollierbar ist, umfasst.

Es ist denkbar, dass das Heizelement eine Widerstandsheizung ist.

Außerdem kann vorgesehen sein, dass das Heizelement eine Strahlungsquelle ist und/oder umfasst, wobei die Strahlungsquelle vorzugsweise eine Infrarotstrahlungsquelle und/oder eine Mikrowellenstrahlungsquelle ist und/oder umfasst.

Des Weiteren ist denkbar, dass das Heizelement ein Peltierelement ist und/oder umfasst. Durch den Einsatz von Peltierelementen kann vorteilhafterweise erreicht werden, dass das Heizelement auch gekühlt werden kann, so dass das Erreichen der Solltemperatur des Heizelementes beschleunigt werden kann.

Möglich ist ferner, dass nach erfolgtem Umschalten auf die Heizelementtemperaturregelung das Heizelement in einem Temperaturbereich von ca. 40°C bis 50°C, insbesondere in einem Temperaturbereich von ca. 42°C bis 46°C betreibbar ist.

Darüber hinaus kann vorgesehen sein, dass das dritte Mittel zum Erfassen eines Parameters, der mit der Flussrate korreliert, ein Erfassungsmittel ist, mittels dessen die Förderleistung des Fördermittels zum Fördern der medizinischen Flüssigkeit durch die Fluidpassage erfassbar ist.

Vorzugsweise ist möglich, dass das Fördermittel eine peristaltische Pumpe, insbesondere eine Schlauchrollenpumpe und/oder eine Verdrängerpumpe ist.

Des Weiteren betrifft die vorliegende Erfindung ein Verfahren zur Erwärmung einer medizinischen Flüssigkeit mit den Merkmalen des Anspruchs 10. Danach ist vorgesehen, dass bei einem Verfahren zum Erwärmen einer medizinischen Flüssigkeit, insbesondere einer Dialysierflüssigkeit durch eine Fluidpassage die medizinische Flüssigkeit gefördert wird, wobei die medizinische Flüssigkeit in der Fluidpassage erwärmt wird, wobei die Fluidtemperatur der medizinischen Flüssigkeit in der Fluidpassage überwacht wird und wobei die Heizelementtemperatur überwacht wird, wobei von einer Fluidtemperaturregelung auf eine Heizelementtemperaturregelung umgeschaltet wird oder umgekehrt, wenn festgestellt wird, dass eine vorher bestimmte Flussratenschwelle der Förderung der medizinischen Flüssigkeit durch die Fluidpassage unter- oder überschritten wird.

Ferner kann vorgesehen sein, dass das Verfahren mit einer Vorrichtung nach einem der Ansprüche 1 bis 9 durchgeführt wird.

Des Weiteren betrifft die vorliegende Erfindung eine Blutbehandlungsvorrichtung mit den Merkmalen des Anspruchs 12. Danach ist vorgesehen, dass eine Blutbehandlungsvorrichtung wenigstens eine Vorrichtung nach einem der Ansprüche 1 bis 9 umfasst.

Die Blutbehandlungsvorrichtung kann insbesondere eine Dialysemaschine oder Peritonealdialysemaschine sein.

Darüber hinaus kann vorgesehen sein, dass mit der Blutbehandlungsvorrichtung des Verfahren nach einem der Ansprüche 10 oder 11 durchführbar ist.

Weitere Einzelheiten und Vorteile der Erfindung sollen nun anhand eines in der Zeichnung dargestellten Ausführungsbeispiels näher erläutert werden. Es zeigen
- Fig. 1:: eine schematische Darstellung der Regelungslogik;
- Fig. 2:: ein erstes Blockschaltbild der erfindungsgemäßen Regelung;
- Fig. 3:: ein weiteres Blockschaltbild der erfindungsgemäßen Regelung in einer zweiten Ausführungsform; und
- Fig. 4:: eine schematische Darstellung der erfindungsgemäßen Vorrichtung zur Erwärmung einer medizinischen Flüssigkeit, hier der Dialysierflüssigkeit.

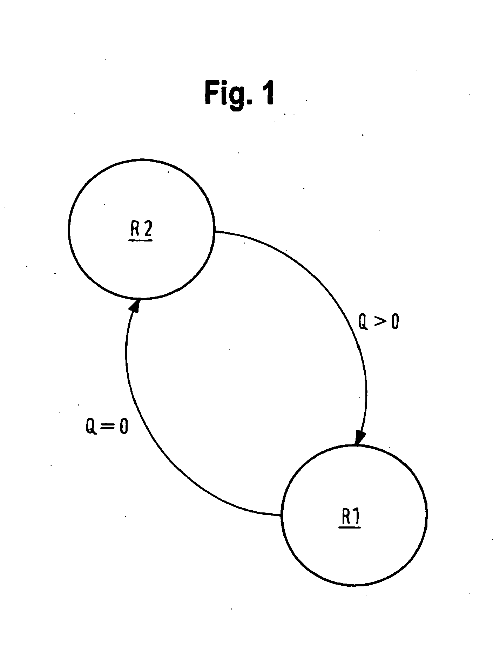

Figur 1 zeigt in schematischer Darstellung die erfindungsgemäße Regelungslogik. So erfolgt bei einer Förderung der Dialysierflüssigkeit, also dem Zustand Q > 0 eine Regelung der Flüssigkeitstemperatur, in Figur 1 mit R1 bezeichnet. Sofern der Zustand Q = 0 eintritt, also keine Förderung der Dialysierflüssigkeit F mehr erfolgt, wird auf eine Regelung der Heizplattentemperatur umgestellt. Dieser Zustand der Regelung der Heizplattentemperatur ist in Figur 1 mit R2 bezeichnet.

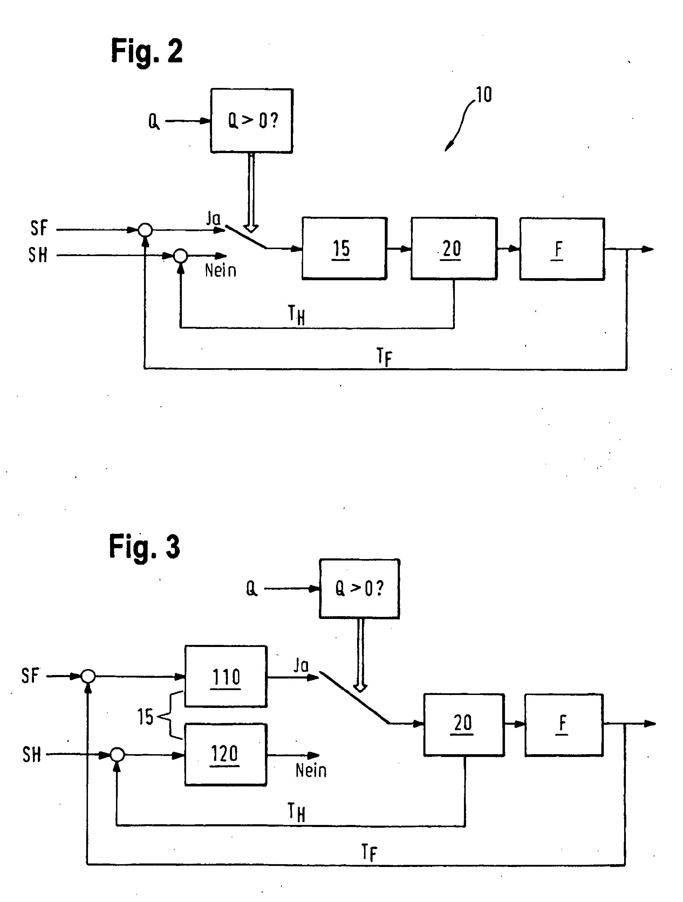

Die Regelung des Heizelements 20 (vgl. Figuren 2 bis 4) orientiert sich somit an den Flusszuständen der Dialysierflüssigkeit. Liegt ein Förderzustand der Dialysierflüssigkeit vor, so nimmt die Regeleinheit die Ausgangsfluidtemperatur als Messgröße, vergleicht diese mit der Vorgabe der Solltemperatur und steuert entsprechend das Heizelement an.

Liegt ein Stillstand der Fluidförderung vor, so nimmt die Regelung die gemessene Temperatur des Heizelements 20 als Messgröße, vergleicht diese mit einer vorgegebenen Solltemperatur des Heizelements 20 und steuert entsprechend das Heizelement 20 an.

Hierbei können weitere Korrekturfaktoren in die Regelung aufgenommen werden.

Als Heizelemente eignen sich insbesondere Widerstandsheizungen, Strahlungsquellen wie Infrarotstrahlungsquellen oder Mikrowellenstrahlungsquellen sowie Peltierelemente.

Bleibt der Durchfluss durch die Heizung aus, so wird die Regelung der Flüssigkeitstemperatur in eine Regelung der Heizplattentemperatur geändert. Diese kann beispielsweise auf einen Wert von 42°C oder 46°C eingestellt sein, um bei wiederanlaufender Flüssigkeitspumpe in jedem Fall einen Alarm durch Übertemperatur der Flüssigkeit zu vermeiden. Sie kann jedoch insbesondere bei der Verwendung von Einwegartikeln auch darüber liegen, da diese Temperatur durch die niedrige Wärmeleitfähigkeit der verwendeten Kunststoffe nicht vollständig am Fluid ankommt.

Durch den Einsatz von Peltierelementen kann vorteilhafterweise erreicht werden, dass die Heizplatte auch gekühlt werden kann, so dass das Erreichen der Solltemperatur der Heizplatte beschleunigt werden kann.

Eine Regelung der Temperatur von Flüssigkeiten in Einwegartikeln oder auch in festverbauten Leitungen ist möglich.

Um den Förderzustand zu überwachen, kann eine direkte Messung des Durchflusses durch die Fluidpassage erfolgen oder es kann eine Verwendung von Vorgabewerten des Betriebs- und/oder Schutzsystems zur Triggerung der Umschaltung der Regelgröße erfolgen.

Figur 2 zeigt schematisch den Regelkreislauf in einer ersten Ausführungsform der Vorrichtung 10 zur Erwärmung einer medizinischen Flüssigkeit F. Wie aus Figur 2 ersichtlich ist, erfolgt eine Überwachung der Flussrate Q und zwar dahingehend, ob der Zustand Q > 0 vorliegt. Die Steuerungs- und/oder Regelungseinheit 15 ist hier durch einen Regler 15 verwirklicht. Sofern dies der Fall ist, erfolgt mittels des Reglers 15 eine Einregelung der Heizung 20 auf die Solltemperatur SF der Flüssigkeit F. Sofern die Bedingung Q > 0 nicht mehr erfüllt ist, erfolgt mittels des Reglers 15 eine Einregelung auf die Solltemperatur SH der Heizplatte 20, so dass mittels der Heizung 20 die Flüssigkeit F nicht überhitzt werden kann. Die Heizplattentemperatur ist mit T_{H} und die Flüssigkeitstemperatur ist mit T_{F} bezeichnet.

Figur 3 zeigt eine weitere Ausgestaltung der erfindungsgemäßen Regelung. Auch hier erfolgt eine Überwachung des Zustands Q > 0. Hier ist jedoch ein gesonderter Regler 110 zur Regelung R1 der Flüssigkeitstemperatur T_{F} und ein gesonderter Regler 120 zur Regelung R2 der Heizplattentemperatur T_{H} vorgesehen, mittels derer jeweils die Heizung 20 zur Temperierung der Flüssigkeit F entsprechend eingeregelt werden kann. Die Regler 110 und 120 bilden hier die Steuerungs- und/oder Regelungseinheit 15. Sofern der Zustand Q > 0 vorliegt, erfolgt eine Regelung mittels des Reglers 110. Sofern der Zustand Q = 0 eintritt, erfolgt eine Regelung der Heizung 20 mittels des Reglers 120, so dass eine Regelung R2 der Heizplattentemperatur T_{H} erfolgt.

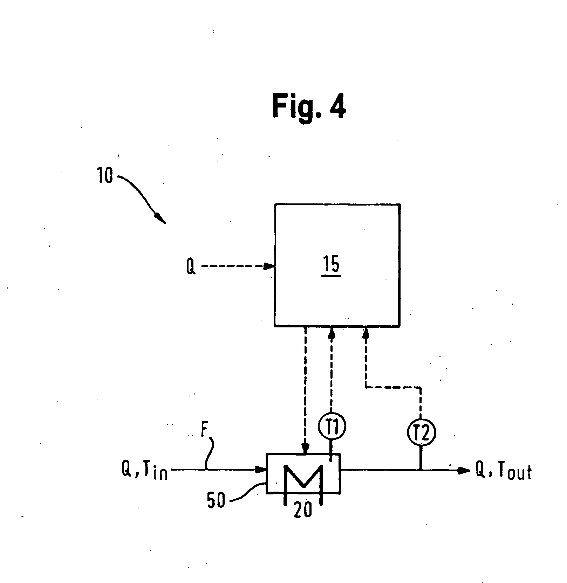

Figur 4 zeigt in schematischer Ansicht die erfindungsgemäße Vorrichtung 10 zur Erwärmung einer Dialysierflüssigkeit F, die mit einer Flussrate Q und einer Eingangstemperatur Tᵢₙ durch die Fluidpassage 50, in der die Heizung 20 angeordnet ist, hindurchströmt. Hierbei erfolgt mittels eines ersten Temperatursensors T1 die Erfassung der Heizplattentemperatur, die an eine Steuerungs- und/oder Regelungseinheit 15 übermittelt wird. Stromabwärts der Fluidpassage 50 erfolgt eine weitere Temperaturerfassung der Dialysierflüssigkeit F mittels eines weiteren Temperatursensors T2, wobei die dort ermittelte Temperatur Tₒᵤₜ ebenfalls an die Steuerungs- und/oder Regelungseinheit 15 übermittelt wird.

Die nicht näher dargestellte Pumpe kann sich dabei stromaufwärts oder stromabwärts der Fluidpassage 50 bzw. der Heizung 20 befinden.

Darüber hinaus kann vorgesehen sein, dass eine Messung der Eingangstemperatur der Flüssigkeit F, also der Temperatur Tᵢₙ erfolgt sowie weiter, dass eine Messung des Durchflusses Q erfolgt.

Eine Messung des Durchflusses Q kann beispielsweise dadurch erfolgen, dass die Drehzahl einer Schlauchrollenpumpe, mittels dessen das Fluid F gefördert wird, ermittelt und an die Steuerungs- und/oder Regelungseinrichtung 15 übertragen wird.

## Patentansprüche

1. Vorrichtung (10) zur Erwärmung einer medizinischen Flüssigkeit (F), umfassend wenigstens eine Fluidpassage (50) zur Förderung der medizinischen Flüssigkeit (F), wenigstens ein Heizelement (20), mittels dessen die medizinische Flüssigkeit (F) in der Fluidpassage (50) erwärmbar ist, wenigstens ein erstes Erfassungsmittel zum Erfassen eines Parameters, der mit einer Flussrate (Q) der medizinischen Flüssigkeit (F) durch die Fluidpassage (50) korreliert, und/oder wenigstens ein zweites Erfassungsmittel zum Erfassen eines Parameters, der einem Sollwert für eine Flussrate (Q) der medizinischen Flüssigkeit (F) durch die Fluidpassage (50) entspricht, wenigstens ein Steuerungs- und/oder Regelungsmittel (15), mittels dessen die Fluidtemperatur des Fluids (F) in der Fluidpassage (50) und/oder die Temperatur des Heizelementes (20) steuer- und/oder regelbar ist,
**gekennzeichnet durch**
ein Umschaltmittel, mittels dessen das Steuerungs- und/oder Regelungsmittel (15) von einer Fluidtemperaturregelung (R1) auf eine Heizelementtemperaturregelung (R2) umschaltbar ist und/oder umgekehrt, wobei das Umschaltmittel derart beschaffen ist, dass eine Umschaltung erfolgt, wenn mittels des Umschaltmittels festgestellt wird, dass ein vorherbestimmter Flussratenschwellwert unter- und/oder überschritten wird.

2. Vorrichtung (10) nach Anspruch 1, **dadurch gekennzeichnet, dass** die Vorrichtung (10) weiter wenigstens ein Fördermittel zum Fördern der medizinischen Flüssigkeit (F) durch die Fluidpassage (50) und/oder wenigstens ein erstes Mittel (T1) zum Erfassen eines Parameters, der mit der Temperatur (T_{H}) des Heizelements (20) korreliert, und/oder wenigstens ein zweites Mittel (T2) zum Erfassen eines Parameters, der mit der Temperatur (T_{F}) der medizinischen Flüssigkeit in der Fluidpassage (50) korreliert, und/oder wenigstens ein drittes Mittel zum Erfassen eines Parameters, der mit der Flussrate (Q) der medizinischen Flüssigkeit (F) durch die Fluidpassage (50) korreliert, und/oder wenigstens ein viertes Mittel zum Erfassen eines Parameters, der einem Sollwert für die Flussrate (Q) der medizinischen Flüssigkeit (F) durch die Fluidpassage (50) entspricht, umfasst.

3. Vorrichtung (10) nach Anspruch 1 oder 2, **dadurch gekennzeichnet, dass** das Steuerungs- und/oder Regelungsmittel (15) wenigstens ein Kontrollmittel, mittels dessen die Fluidtemperatur (T_{F}) anhand eines Parameters, der mit der Fluidtemperatur (T_{F}) korreliert, und/oder wenigstens ein zweites Kontrollmittel, mittels dessen die Heizelementtemperatur (T_{H}) anhand eines Parameters, der mit der Temperatur (T_{H}) des Heizelements (20) korreliert, kontrollierbar ist, umfasst.

4. Vorrichtung (10) nach einem der vorhergehenden Ansprüche, **dadurch gekennzeichnet, dass** das Heizelement (20) eine Widerstandsheizung ist.

5. Vorrichtung (10) nach einem der vorhergehenden Ansprüche, **dadurch gekennzeichnet, dass** das Heizelement (20) eine Strahlungsquelle ist und/oder umfasst, wobei die Strahlungsquelle vorzugsweise eine Infrarotstrahlungsquelle und/oder eine Mikrowellenstrahlungsquelle ist und/oder umfasst.

6. Vorrichtung (10) nach einem der vorhergehenden Ansprüche, **dadurch gekennzeichnet, dass** das Heizelement (20) ein Peltierelement ist und/oder umfasst.

7. Vorrichtung (10) nach einem der vorhergehenden Ansprüche, **dadurch gekennzeichnet, dass** nach erfolgtem Umschalten auf die Heizelementtemperaturregelung (R2) das Heizelement (20) in einem Temperaturbereich von ca. 37°C bis 60°C, insbesondere in einem Temperaturbereich von ca. 42°C bis 46°C betreibbar ist.

8. Vorrichtung (10) nach einem der vorhergehenden Ansprüche, **dadurch gekennzeichnet, dass** das dritte Mittel zum Erfassen eines Parameters, der mit der Flussrate (Q) korreliert, ein Erfassungsmittel ist, mittels dessen die Förderleistung des Fördermittels zum Fördern der medizinischen Flüssigkeit durch die Fluidpassage (50) erfassbar ist.

9. Vorrichtung (10) nach einem der vorhergehenden Ansprüche, **dadurch gekennzeichnet, dass** das Fördermittel eine peristaltische Pumpe, insbesondere eine Schlauchrollenpumpe und/oder eine Verdrängerpumpe ist.

10. Vorrichtung nach einem der vorangegangenen Ansprüche, wobei es sich bei der medizinischen Flüssigkeit um eine Dialyseflüssigkeit (F) handelt.

11. Verfahren zum Erwärmen einer medizinischen Flüssigkeit (F), wobei durch eine Fluidpassage (50) die medizinische Flüssigkeit (F) gefördert wird, wobei die medizinische Flüssigkeit (F) in der Fluidpassage (50) erwärmt wird, wobei die Fluidtemperatur (T_{F}) der medizinischen Flüssigkeit (F) in der Fluidpassage (50) überwacht wird und wobei die Heizelementtemperatur (T_{H}) überwacht wird, wobei von einer Fluidtemperaturregelung (R1) auf eine Heizelementtemperaturregelung (R2) umgeschaltet wird oder umgekehrt, wenn festgestellt wird, dass eine vorher bestimmte Flussrätenschwelle der Förderung der medizinischen Flüssigkeit (F) durch die Fluidpassage (50) unter- oder überschritten wird.

12. Verfahren nach Anspruch 11, **dadurch gekennzeichnet, dass** das Verfahren mit einer Vorrichtung (10) nach einem der Ansprüche 1 bis 9 durchgeführt wird.

13. Verfahren nach Anspruch 12, wobei es sich bei der medizinischen Flüssigkeit (F) um eine Dialyseflüssigkeit handelt.

14. Blutbehandlungsvorrichtung, insbesondere Dialysemaschine oder Peritonealdialysemaschine, umfassend wenigstens eine Vorrichtung (10) nach einem der Ansprüche 1 bis 10.

15. Blutbehandlungsvorrichtung nach Anspruch 14, **dadurch gekennzeichnet, dass** mit der Blutbehandlungsvorrichtuhg des Verfahren nach einem der Ansprüche 11 bis 13 durchführbar ist.

## Claims

1. An apparatus (10) for heating a medical fluid (F), comprising at least one fluid passage (50) for delivering the medical fluid (F), at least one heating element (20) by means of which the medical fluid (F) in the fluid passage (50) can be heated, at least one first detection means for detecting a parameter which correlates with the flow rate (Q) of the medical fluid (F) through the fluid passage (50), and/or at least one second detection means for detecting a parameter which corresponds to a set point for the flow rate (Q) of the medical fluid (F) through the fluid passage (50), at least one control and/or regulating means (15) by means of which the fluid temperature of the fluid (F) in the fluid passage (50) and/or the temperature of the heating element (20) can be controlled and/or regulated, **characterized by**
a switching means by means of which the control and/or regulating means (15) can be switched from a fluid temperature control (R1) to a heating element temperature control (R2) and/or vice versa, wherein the switching means is designed such that switching is effected when it is detected by means of the switching means that the flow rate falls below and/or exceeds a predetermined flow rate threshold value.

2. The apparatus (10) according to claim 1, **characterized in that** the apparatus (10) furthermore comprises at least one delivering means for delivering the medical fluid (F) through the fluid passage (50) and/or at least one first means (T1) for detecting a parameter which correlates with the temperature (T_{H}) of the heating element (20) and/or at least one second means (T2) for detecting a parameter which correlates with the temperature (T_{F}) of the medical fluid in the fluid passage (50) and/or at least one third means for detecting a parameter which correlates with the flow rate (Q) of the medical fluid (F) through the fluid passage (50) and/or at least one fourth means for detecting a parameter which corresponds to a set point for the flow rate (Q) of the medical fluid (F) through the fluid passage (50).

3. The apparatus (10) according to claim 1 or 2, **characterized in that** the control and/or regulating means (15) comprises at least one checking means by means of which the fluid temperature (T_{F}) can be checked with reference to a parameter which correlates with the fluid temperature (T_{F}) and/or at least one second checking means by means of which the heating element temperature (T_{H}) can be checked with reference to a parameter which correlates with the temperature (T_{H}) of the heating element (20).

4. The apparatus (10) according to any of the preceding claims, **characterized in that** the heating element (20) is a resistance heater.

5. The apparatus (10) according to any of the preceding claims, **characterized in that** the heating element (20) is and/or comprises a radiation source, wherein the radiation source preferably is and/or comprises an infrared radiation source and/or a microwave radiation source.

6. The apparatus (10) according to any of the preceding claims, **characterized in that** the heating element (20) is and/or comprises a Peltier element.

7. The apparatus (10) according to any of the preceding claims, **characterized in that** after having switched to the heating element temperature control (R2), the heating element (20) is operable in a temperature range from about 37°C to 60°C, in particular in a temperature range from about 42°C to 46°C.

8. The apparatus (10) according to any of the preceding claims, **characterized in that** the third means for detecting a parameter which correlates with the flow rate (Q) is a detection means by means of which the delivery rate of the delivering means for delivering the medical fluid through the fluid passage (50) can be detected.

9. The apparatus (10) according to any of the preceding claims, **characterized in that** the delivering means is a peristaltic pump, in particular a peristaltic hose pump and/or a positive-displacement pump.

10. The apparatus according to any of the preceding claims, wherein the medical fluid is a dialysis fluid (F).

11. A method for heating a medical fluid (F), wherein the medical fluid (F) is delivered through a fluid passage (50), wherein the medical fluid (F) is heated in the fluid passage (50), wherein the fluid temperature (T_{F}) of the medical fluid (F) in the fluid passage (50) is monitored, and wherein the heating element temperature (T_{H}) is monitored, wherein switching is effected from a fluid temperature control (R1) to a heating element temperature control (R2) or vice versa, when it is detected that the flow rate falls below or exceeds a predetermined flow rate threshold of the delivery of the medical fluid (F) through the fluid passage (50).

12. The method according to claim 11, **characterized in that** the method is carried out with an apparatus (10) according to any of claims 1 to 9.

13. The method according to claim 12, wherein the medical fluid (F) is a dialysis fluid.

14. A blood treatment device, in particular a dialysis machine or peritoneal dialysis machine, comprising at least one apparatus (10) according to any of claims 1 to 10.

15. The blood treatment device according to claim 14, **characterized in that** by means of the blood treatment device the method according to any of claims 11 to 13 can be carried out.

## Revendications

1. Dispositif (10) pour chauffer un liquide médical (F), comprenant au moins un passage de fluide (50) pour transporter le liquide médical (F), au moins un élément de chauffage (20) au moyen duquel le liquide médical (F) peut être chauffé dans le passage de fluide (50), au moins un premier moyen de détection pour détecter un paramètre qui est en corrélation avec un débit (Q) du liquide médical (F), et/ou au moins un deuxième moyen de détection pour détecter un paramètre qui correspond à une valeur de consigne pour un débit (Q) du liquide médical (F) à travers le passage de fluide (50), au moins un moyen de commande et/ou de réglage (15) grâce auquel la température de fluide du fluide (F) dans le passage de fluide (50) et/ou la température de l'élément de chauffage (20) peut être commandé et/ou réglé,
**caractérisé par**
un moyen de commutation grâce auquel le moyen de commande et/ou de réglage (15) peut être commuté depuis un réglage de température de fluide (R1) sur un réglage de température d'élément de chauffage (R2) et/ou inversement, le moyen de commutation étant conçu de telle sorte qu'une commutation se produit lorsque grâce au moyen de commutation, il est constaté qu'une valeur seuil de débit prédéterminée n'est pas atteinte et/ou est dépassée.

2. Dispositif (10) selon la revendication 1, **caractérisé en ce que** le dispositif (10) comprend en outre au moins un moyen de transport pour transporter le liquide médical (F) à travers le passage de fluide (50) et/ou au moins un premier moyen (T1) pour détecter un paramètre qui est en corrélation avec la température (T_{H}) de l'élément de chauffage (20), et/ou au moins un deuxième moyen (T2) pour détecter un paramètre qui est en corrélation avec la température (T_{F}) du liquide médical dans le passage de fluide (50), et/ou au moins un troisième moyen pour détecter un paramètre qui est en corrélation avec le débit (Q) du liquide médical (F) à travers le passage de fluide (50), et/ou au moins un quatrième moyen pour détecter un paramètre qui correspond à une valeur de consigne pour le débit (Q) du liquide médical (F) à travers le passage de fluide (50).

3. Dispositif (10) selon la revendication 1 ou 2, **caractérisé en ce que** le moyen de commande et/ou de réglage (15) comporte au moins un moyen de contrôle grâce auquel la température de fluide (T_{F}) peut être contrôlée à l'aide d'un paramètre qui est en corrélation avec la température de fluide (T_{F}) et/ou au moins un deuxième moyen de contrôle grâce auquel la température d'élément de chauffage (T_{H}) peut être contrôlée à l'aide d'un paramètre qui est en corrélation avec la température (T_{F}) de l'élément de chauffage (20).

4. Dispositif (10) selon l'une des revendications précédentes, **caractérisé en ce que** l'élément de chauffage (20) est un chauffage par résistance.

5. Dispositif (10) selon l'une des revendications précédentes, **caractérisé en ce que** l'élément de chauffage (20) est et/ou comprend une source de rayonnement, la source de rayonnement étant et/ou comprenant de préférence une source de rayonnement infrarouge et/ou une source de rayonnement micro-ondes.

6. Dispositif (10) selon l'une des revendications précédentes, **caractérisé en ce que** l'élément de chauffage (20) est et/ou comprend un élément Peltier.

7. Dispositif (10) selon l'une des revendications précédentes, **caractérisé en ce qu'**après avoir été commuté sur le réglage de température de l'élément de chauffage (R2), l'élément de chauffage (20) peut fonctionner dans une plage de température d'environ 37°C à 60°C, en particulier dans une plage de température d'environ 42°C à 46 °C.

8. Dispositif (10) selon l'une des revendications précédentes, **caractérisé en ce que** le troisième moyen pour détecter un paramètre qui est en corrélation avec le débit (Q) est un moyen de détection grâce auquel la capacité de transport du moyen de transport pour transporter le liquide médical à travers le passage de fluide (50) peut être détectée.

9. Dispositif (10) selon l'une des revendications précédentes, **caractérisé en ce que** le moyen de transport est une pompe péristaltique, en particulier une pompe à rouleau tubulaire et/ou une pompe volumétrique.

10. Dispositif selon l'une des revendications précédentes, le liquide médical étant un liquide de dialyse (F).

11. Procédé pour chauffer un liquide médical (F), dans lequel le liquide médical (F) est transporté à travers un passage de fluide (50), ledit liquide médical (F) étant chauffé dans le passage de fluide (50), la température de fluide (T_{F}) dudit liquide médical (F) dans le passage de fluide (50) étant surveillée, et la température de l'élément de chauffage (T_{H}) étant surveillée, une commutation depuis un réglage de température de fluide (R1) sur un réglage de température de l'élément de chauffage (R2) ou inversement étant effectuée s'il est constaté qu'un seuil de débit, déterminé auparavant, du transport du liquide médical (F) à travers le passage de fluide (50) n'est pas atteint et/ou est dépassé.

12. Procédé selon la revendication 11, **caractérisé en ce que** le procédé est mis en oeuvre avec un dispositif (10) selon l'une des revendications 1 à 9.

13. Procédé selon la revendication 12, dans lequel le liquide médical est un liquide de dialyse (F).

14. Dispositif de traitement de sang, en particulier une machine pour dialyse ou une machine pour dialyse péritonéale, comprenant au moins un dispositif (10) selon l'une des revendications 1 à 10.

15. Dispositif de traitement de sang selon la revendication 14, **caractérisé en ce que** le procédé selon l'une des revendications 11 à 13 peut être mis en oeuvre avec le dispositif de traitement de sang.
